# EUROPÄISCHE PATENTSCHRIFT

(11) **EP 2 655 608 B1**
(45) Veröffentlichungstag und Bekanntmachung des Hinweises auf die Patenterteilung: **29.06.2016**
(21) Anmeldenummer: 11808130.6
(22) Anmeldetag: 22.12.2011
(51) Int. Cl.: C12N 9/10, C12N 9/12, C12N 9/90, C12N 15/80, C12P 19/26

(54) **VERFAHREN UND MITTEL ZUR HERSTELLUNG VON N-ACETYLNEURAMINSÄURE (NEUNAC)**
PROCESS AND MEANS FOR THE PRODUCTION OF N-ACETYLNEURAMINIC ACID (NEUNAC)
PROCEDE ET MOYEN POUR LA PRODUCTION D'ACIDE N-ACETYLNEURAMINIQUE (NEUNAC)

(30) Priorität: 22.12.2010 AT 21112010
(43) Veröffentlichungstag der Anmeldung: 30.10.2013
(73) Patentinhaber: Technische Universität Wien, 1040 Wien (AT)
(72) Erfinder: MACH-AIGNER, Astrid, A-3011 Untertullnerbach (AT); MACH, Robert, A-3011 Untertullnerbach (AT); STEIGER, Matthias, G., A-1180 Wien (AT)
(74) Vertreter: Sonn & Partner Patentanwälte
(86) Internationale Anmeldenummer: PCT/AT2011/000510
(87) Internationale Veröffentlichungsnummer: WO 2012/083329

(56) Entgegenhaltungen:
- EP-A1- 1 154 018
- EP-A1- 1 484 406
- EP-A2- 1 081 230
- WO-A2-2005/090552
- MATTHIAS G STEIGER ET AL: "Synthesis of an antiviral drug precursor from chitin using a saprophyte as a whole-cell catalyst", MICROBIAL CELL FACTORIES, Bd. 10, Nr. 1, 5. Dezember 2011 (2011-12-05), Seite 102, XP55025863, ISSN: 1475-2859, DOI: 10.1186/1475-2859-10-102
- BLUME A ET AL: "UDP-N-acetylglucosamine 2-epimerase/N-acetylmannosamine kinase, functionally expressed in and purified from Escherichia coli, yeast, and insect cells", PROTEIN EXPRESSION AND PURIFICATION, ACADEMIC PRESS, SAN DIEGO, CA, Bd. 35, Nr. 2, 1. Juni 2004 (2004-06-01), Seiten 387-396, XP004506578, ISSN: 1046-5928, DOI: 10.1016/J.PEP.2004.02.013
- KAZUHIKO TABATA ET AL: "Production of N-acetyl-d-neuraminic acid by coupling bacteria expressing N-acetyl-d-glucosamine 2-epimerase and N-acetyl-d-neuraminic acid synthetase", ENZYME AND MICROBIAL TECHNOLOGY, Bd. 30, Nr. 3, 1. März 2002 (2002-03-01), Seiten 327-333, XP55026513, ISSN: 0141-0229, DOI: 10.1016/S0141-0229(01)00515-4

## Beschreibung

Die vorliegende Erfindung betrifft Verfahren und Mittel zur Herstellung von N-Acetylneuraminsäure (NeuNAc).

N-Acetylneuraminsäure (NeuNAc) gehört zu den Sialinsäuren. In Säugetieren werden Sialinsäuren üblicherweise als terminaler Rest von Zucker-Konjugaten an der Zelloberfläche gefunden. Aufgrund ihrer endständigen Position und der negativen Carboxylierungsfunktion spielen Sialinsäuren eine wichtige Rolle bei der zellulären Erkennung und bei Adhäsionsprozessen.

Derivate von NeuNAc werden als Neuraminidase-Inhibitoren zur Behandlung von viralen Infektionen, wie Influenza, eingesetzt. NeuNAc dient dabei als ein Edukt für die Herstellung derartiger Medikamente, wie z.B. Oseltamivir und Zanamivir. NeuNAc kann aus entsprechenden Rohstoffen, wie Milch und Eiern, extrahiert oder auch chemisch synthetisiert werden. Derzeit wird NeuNAc ausschließlich aus Rohstoffen wie N-Acetylglucosamin hergestellt, wobei ein Teil der bekannten Verfahren enzymatisch katalysierte Schritte umfasst.

In EP 1 154 018 A1 wird eine N-Acetylglukosamin 2-Epimerase mit einer spezifischen Aminosäuresequenz beschrieben. Gemäß dieser europäischen Patentanmeldung kann diese Epimerase rekombinant in verschiedensten Wirtszellen, unter anderem in Hefen, hergestellt werden. Dabei können Expressionsvektoren verwendet werden, die einen Promoter aufweisen, der funktionell an ein für N-Acetylglukosamin 2-Epimerase kodierenden Nukleinsäuremolekül gebunden ist.

In der EP 1 484 406 A1 wird ein Verfahren zur Herstellung von N-Acetylneuraminsäure beschrieben. Dabei werden verschiedenste induzierbare Promotoren erwähnt, die in der Lage sind, die Expression der Enzyme, die bei der Herstellung von N-Acetylneuraminsäure benötigt werden, zu kontrollieren.

In der WO 94/29476 wird ein Verfahren zur Herstellung von NeuNAc beschrieben, bei dem zunächst N-Acetyl-D-Glukosamin zu N-Acetyl-D-Mannosamin durch Epimerisierung umgewandelt wird. Das Produkt dieses ersten Schritts wird anschließend mit Pyruvat und einer NeuNAc-Aldolase zu NeuNAc umgesetzt.

In der US 7,579,175 und der EP 1 081 230 A2 wird ein Verfahren zur Herstellung von NeuNAc beschrieben, bei dem Mikroorganismen mit einer NeuNAc Synthetase-Aktivität und Bakterien, wie beispielsweise E. coli, die in der Lage sind Phosphophenolbenztraubensäure zu synthetisieren, in einem Medium kultiviert werden, in dem sich N-Acetylmannosamin und Glukose oder Fruktose befindet.

Alternativ zu den zuvor genannten Verfahren wird in der EP 0 578 825 ein Verfahren zur Herstellung von NeuNAc geoffenbart, bei dem N-Acetylglukosamin und Benztraubensäure mit N-Acetylneuraminsäure-Lyase umgesetzt wird.

Die WO 2005/090552 A2 offenbart die Expression von UDP-N-Acetylglucosamin-2-Epimerase in verschiedensten Wirtszellen.

Blume A et al., Protein Expression and Purification, Bd.35, Nr. 2 (2004): 387-396, beschreibt die rekombinante Expression von UDP-N-Acetylglucosamin-2-Epimerase in E.coli, Hefe- und Insektenzellen

Kazuhiko Tabata et al., Enzyme and Microbial Technology, Bd. 30, Nr. 3 (2002): 327-333, beschreibt die Herstellung von N-Acetyl-D-Neuraminsäure unter Zuhilfenahme von in unterschiedlichen E.coli Zellen rekombinant hergestellten Enzyme N-Acetylglucosamin-2-Epimerase und N-Acetyl-D-Neuraminsäure-Synthase.

Der Nachteil der zuvor beschriebenen Verfahren liegt darin, dass es sich zumeist um Gleichgewichtsreaktionen handelt, bei denen ein Überschuss an Pyruvat eingesetzt werden muss, um die Gleichgewichtsreaktion zur NeuNAc zu verschieben. Des Weiteren ist das in diesen Reaktionen verwendete N-Acetylglukosamin zu teuer, um kostengünstig NeuNAc herzustellen. Es ist somit Aufgabe der vorliegenden Erfindung ein Verfahren zur Verfügung zu stellen, welches die zuvor genannten Nachteile nicht aufweist.

Die vorliegende Erfindung betrifft ein isoliertes a) Nukleinsäuremolekül umfassend mindestens einen in Pilzzellen der Gattung Trichoderma aktiven Promoter an dem jeweils eine Nukleinsäuresequenz kodierend für eine N-Acetylglukosamin-2-Epimerase (EC 5.1.3.8) und eine N-Acetylneuraminsäure-Synthase (EC 2.5.1.56) operativ gebunden ist, oder b) Set mit zwei Nukleinsäuremolekülen, wobei ein Molekül für die N-Acetylglukosamin-2-Epimerase (EC 5.1.3.8) und ein Molekül für die N-Acetylneuraminsäure-Synthase (EC 2.5.1.56) kodiert, jeweils umfassend mindestens einen in Pilzzellen der Gattung Trichoderma aktiven Promoter operativ an die N-Acetylglukosamin-2-Epimerase oder N-Acetylneuraminsäure-Synthase kodierende Sequenz gebunden; wobei gemäß a) oder b) der mindestens eine in Pilzzellen aktive Promoter ein konstitutiver Promoter ist.

Es hat sich erfindungsgemäß herausgestellt, dass NeuNAc in einfacher und effizienter Weise in einer Pilzzelle der Gattung Trichoderma hergestellt werden kann, wenn diese Pilzzelle in der Lage ist N-Acetylglukosamin-2-Epimerase und N-Acetylneuraminsäure-Synthase konstitutiv zu exprimieren. Die dabei eingesetzte Pilzzelle sollte auch in der Lage sein, ausreichend N-Acetyl-D-Glukosamin bereit zu stellen, um daraus mithilfe der N-Acetylglukosamin-2-Epimerase N-Acetyl-D-Mannosamin herzustellen, welches schließlich mithilfe der N-Acetylneuraminsäure-Synthase NeuNAc synthetisiert. Selbstverständlich wäre es auch möglich, Pilzzellen zu verwenden, die nicht in der Lage sind N-Acetyl-D-Glukosamin bereitzustellen. In einem derartigen Fall müsste N-Acetyl-D-Glukosamin dem Kultivierungsmedium zugesetzt werden oder es werden Organismen verwendet, die in der Lage sind N-Acetyl-D-Glukosamin an das Medium abzugeben.

Da natürlich vorkommende Pilzzellen nicht in der Lage sind N-Acetylglukosamin-2-Epimerase und N-Acetylneuraminsäure-Synthase zu exprimieren müssen in die verwendeten Pilzzellen entsprechende Nukleinsäuremoleküle eingebracht werden, welche die entsprechenden Nukleinsäuresequenzen umfassen. Das einzubringende Nukleinsäuremolekül kann dabei die Nukleinsäuresequenzen beider Enzyme umfassen. Alternativ dazu können auch zwei Nukleinsäuremoleküle in die Pilzzelle eingebracht werden, wobei ein Molekül für die N-Acetylglukosamin-2-Epimerase und ein Molekül für die N-Acetylneuraminsäure-Synthase kodiert. Beide Moleküle müssen jedoch konstitutiv in der Wirtszelle exprimiert werden.

Um eine konstitutive Expression der beiden Enzyme zu ermöglichen, sind deren kodierenden Nukleinsäuresequenzen operativ an einen in Pilzzellen konstitutiv wirkenden Promoter gebunden.

Erfindungsgemäß bedeutet "operativ daran gebunden", dass die Nukleotidsequenz kodierend für die erfindungsgemäßen Enzyme derart an die Regulationssequenz(en) gebunden ist, dass die Expression der Nukleotidsequenz möglich ist und die beiden Sequenzen derart aneinander gebunden sind, dass sie die vorhergesagte, der Sequenz zugeschriebene Funktion erfüllen. Eine Nukleinsäure ist "operativ gebunden", wenn sie in eine funktionelle Beziehung mit einer anderen Nukleinsäuresequenz gebracht wird. Ein Promotor ist somit operativ an eine kodierende Sequenz gebunden, wenn er die Transkription der Sequenz beeinflusst. Das Binden wird durch Ligation an zweckdienlichen Restriktionsstellen erzielt. Wenn solche Stellen nicht vorliegen, werden synthetische Oligonukleotidadaptoren gemäß herkömmlicher Praxis verwendet.

Ein "konstitutiver Promoter" ist ein Promotor, der es einem Gen oder Operon ermöglicht, kontinuierlich in einer Zelle exprimiert zu werden. Ein "konstitutiver Promoter" ist in den meisten Phasen des Wachstums transkriptionell aktiv. Im Gegensatz dazu kann die Expressionsrate von Genen oder Operons, welche operativ an "induzierbare Promotoren" gebunden sind, gezielt geregelt werden, so dass unter bestimmten Bedingungen die Transkription gänzlich herunterreguliert wird und unter anderen, vorzugsweise extrinsischen, Bedingungen hochreguliert wird.

N-Acetyl-D-Glukosamin-2-Epimerase bzw. N-Acetylglukosamin-2-Epimerase (EC 5.1.3.8) katalysiert die Reaktion von N-Acetylglukosamin zu N-Acetylmannosamin. Die kodierende Nukleinsäuresequenz dieses Enzyms ist u.a. von Säugetieren und Bakterien, wie Cyanobakterien, beschrieben, in denen diese Enzyme exprimiert werden. Die entsprechenden kodierenden Nukleinsäuresequenzen dieser Enzyme können erfindunsgemäß eingesetzt werden. Um die Expression in Pilzzellen zu verbessern, ist es möglich aus einer Aminosäuresequenz der Epimerase Kodon-optimierte Nukleinsäuresequenzen zu erzeugen, die schließlich im erfindungsgemäßen Nukleinsäuremolekül eingesetzt werden. Es ist besonders bevorzugt die N-Acetylglukosamin-2-Epimerase aus Anabaena sp. (GenBank ABG57042) im erfindungsgemäßen Nukleinsäuremolekül vorzusehen und in Pilzzellen zu exprimieren.

Um die Expression der zuvor genannten Enzyme zu verbessern, ist es von Vorteil die Nukleinsäuresequenzen bezüglich deren Kodonhäufigkeit in einer Wirtszelle, in der diese eingebracht werden, zu optimieren. Die Kodon-Optimierung wurde anhand der Information über die Häufigkeit der Kodons, z.B., in *Trichoderma reesei* durchgeführt. Diese Information kann aus der "Codon Usage Database" abgerufen werden und die Kodonverwendung von Trichoderma reseei ist in Tabelle 1 angeführt.

**Tabelle 1: Kodonverwendung bei Hypocrea jecorina (Trichoderma reseei), z.B. basierend auf der Analyse von 118 CDS's (54050 codons) (Quelle: http://www.kazusa.or.jp/codon/cgi-bin/showcodon.cgi?species=51453)**

| Felder: [Triplet] [Vorkommenshäufigkeit per 1000] ([Anzahl]) | | | | | | | | | | | |
|---|---|---|---|---|---|---|---|---|---|---|---|
| UUU | 13.3( | 719) | UCU | 10.7( | 580) | UAU | 8.6( | 463) | UGU | 3.0( | 164) |
| UUC | 24.3( | 1311) | UCC | 20.4( | 1101) | UAC | 27.5( | 1486) | UGC | 10.6( | 575) |
| UUA | 0.7( | 39) | UCA | 6.4( | 345) | UAA | 0.9( | 50) | UGA | 0.5( | 29) |
| UUG | 7.9( | 428) | UCG | 16.0( | 866) | UAG | 0.7( | 39) | UGG | 16.7( | 904) |
| | | | | | | | | | | | |
| CUU | 9.4( | 510) | CCU | 12.3( | 663) | CAU | 5.3( | 285) | CGU | 5.5( | 295) |
| CUC | 29.9( | 1617) | CCC | 25.4( | 1375) | CAC | 17.7( | 957) | CGC | 17.4( | 939) |
| CUA | 2.3( | 125) | CCA | 6.9( | 371) | CAA | 8.7( | 469) | CGA | 7.3( | 394) |
| | 27.3( | 1473) | CCG | 11.9( | 641) | CAG | 31.9( | 1725) | CGG | 5.6( | 300) |
| | | | | | | | | | | | |
| AUU | 16.0( | 866) | ACU | 10.7( | 578) | AAU | 8.3( | 447) | AGU | 3.9( | 213) |
| AUC | 31.0( | 1676) | ACC | 27.2( | 1470) | AAC | 38.4( | 2077) | AGC | 22.2( | 1202) |
| AUA | 2.1( | 115) | ACA | 7.5( | 405) | AAA | 4.6( | 250) | AGA | 2.9( | 158) |
| | 20.1( | 1085) | ACG | 18.3( | 987) | AAG | 42.4( | 2293) | AGG | 5.2( | 279) |
| | | | | | | | | | | | |
| GUU | 11.2( | 608) | GCU | 18.0( | 972) | GAU | 15.8( | 854) | GGU | 14.0( | 754) |
| GUC | 36.9( | 1992) | GCC | 48.0( | 2596) | GAC | 41.0( | 2214) | GGC | 51.0( | 2758) |
| GUA | 2.4( | 131) | GCA | 10.5( | 566) | GAA | 10.2( | 551) | GGA | 13.3( | 720) |
| GUG | 14.8( | 800) | GCG | 14.2( | 765) | GAG | 38.0( | 2052) | GGG | 7.0( | 378) |

N-Acetylneuraminsäure-Synthase (EC 2.5.1.56) katalysiert die Reaktion von N-Acetylmannosamin zu NeuNAc. Bei dieser Reaktion wird zudem Phosphoenolpyruvat und Wasser als Co-Substrat umgesetzt. N-Acetylneuraminsäure-Synthase wird in Bakterien, wie E. coli, Campylobacter jejuni und Neisseria meningitidis, exprimiert. Die entsprechenden Nuklein- uns Aminosäuresequenzen sind somit hinreichend bekannt bzw. identifizierbar. Aus den bekannten Sequenzen ist es möglich Kodon-optimierte Nukleinsäuresequenzen abzuleiten, die besonders gut in Pilzzellen transkribiert bzw. translatiert werden. Es ist besonders bevorzugt N-Acetylneuraminsäure-Synthase aus Campylobacter jejuni (e.g. C. jejuni NCTC11168) im erfindungsgemäßen Nukleinsäuremolekül vorzusehen und in Pilzzellen zu exprimieren.

Gemäß einer besonders bevorzugten Ausführungsform der vorliegenden Erfindung wird die N-Acetyl-D-Glukosamin-2-Epimerase durch die folgende Nukleinsäuresequenz kodiert:

atggcaagaacctccaggccctggcccagctctacaagaacgccctcctca-
acgacgtcctgcccttctgggagaaccacagcctcgacagcgagggcggctacttcac-
ctgcctcgaccgccagggcaaggtctacgacaccgacaagttcatctggctccagaac-
cgccaggtctggaccttcagcatgctctgcaaccagctggagaagcgcgagaactggctcaa-
gatcgcccgcaacggcgccaagttcctcgcccagcacggccgcgacgacgagggcaactgg-
tactttgccctgacccgcggcggcgagcctctggtccagccctacaacatcttca-

gcgactgcttcgccgccatggccttcagccagtacgccctcgccagcggcgag-
gagtgggccaaggacgtcgccatgcaggcctacaacaacgtcctccgccgcaaggacaac-
cccaagggcaagtacaccaagacctaccccggcacccgccccat-
gaaggccctggctgtccccatgatcctcgccaacctcaccctggagatg-
gagtggctcctcccccaggagaccctggagaacgtcctcgccgccaccgtccaggaggtcat-
gggcgacttcctcgaccaggagcagggcctcatg-
tacgagaacgtcgcccccgacggcagccacatcgactgcttcgagggccgcctcatcaac-
cccggccacggcatcgaggccatgtggttcatcatg-
gacatcgcccgccgcaagaacgacagcaagaccatcaaccaggccgtcgacgtcgtcctca-
acatcctcaacttcgcctgggacaacgagtacggcggcctctactacttcatg-
gacgccgccggccacccccccagcagctggagtgggaccagaagctctggtgggtccacctg-
gagagcctcgtcgccctcgccatgggctaccgcctcaccggccgcgacgcctgctgggcctgg-
tatcagaagatgcacgactacagctggcagcacttcgccgaccctgagtacggcgagtggtt-
cggctacctcaaccgccgaggcgaggtcctcctcaacctcaagggcggcaagtg-
gaagggctgcttccacgtcccccgcgccatgtacctctgctggcagcagttcgaggccctca-
gctaa

Gemäß einer weiteren bevorzugten Ausführungsform der vorliegenden Erfindung wird die N-Acetylneuraminsäure-Synthase durch die folgende Nukleinsäuresequenz kodiert:

atgcagatcaagatcgacaagctcaccatcagccagaagaacccccctcatcatccccga-
gatcggcatcaaccacaacggcagcctggagatcgccaagctcat-
ggtcgacgccgccaagcgagccggcgccaagatcatcaagcaccagacccacatcgtcgag-
gacgagatgagccaggaggccaagaacgtcatccccggcaacgccaacatcagcatctacga-
gatcatggagcagtgcgccctcaactacaaggacgagctggccctcaaggag-
tacgtcgagaagcagggcctcgtctacctcagcacccccttcagccgcgccgccgccaac-
cgcctggaggacatgggcgtcagcgcctacaagatcggcagcggcgagtgcaacaactac-
cccctgatcaagcacatcgcccagttcaagaagcccatgatcatcagcaccggcat-
gaacagcatcgagagcatcaagcccaccgtcaagatcctccgcgactacgagatccccctt-
cgtcctcctgcacaccaccaacctctaccccacccccagccac-
ctcgtccgcctccaggccatgctggagctgtacaaggagttcaactgcctctacggcctca-
gcgaccacacgacgaacaacctcgcctgcatcggcgccatcgccctcggcgccagcgtcctg-
gagcgccacttcaccgacaccatggaccgcaagggccccgacatcgtctgcagcatg-
gacgagagcaccctcaaggacctcatcaaccagacccaggagat-
ggtcctcctccgcggcgacaacaacaagaacccccctgaaggaggagcaggtcacca-
tcgacttcgccttcgccagcgtcgtcagcatcaaggacatcaagaagggcgagatcctca-
gcatggacaacatctgggtcaagcgccccagcaagggcggcatcagcgccaaggactt-
cgaggccatcctcggcaagcgcgccaagaaggacatcaagaacaacatccagctcacctgg-
gacgacttcgagtaa

Die erfindungsgemäßen Pilzzellen gehören der Gattung Trichoderma an.

Pilzzellen der Gattung Trichoderma eignen sich besonders gut zur Biosynthese von NeuNAc, da die Mitglieder dieser Gattung in der Lage sind ausreichend N-Acetyl-D-Glukosamin zur Verfügung zu stellen.

Gemäß einer besonders bevorzugten Ausführungsform der vorliegenden Erfindung sind die Pilzzellen Trichoderma reesei Zellen.

Gemäß einer bevorzugten Ausführungsform der vorliegenden Erfindung ist der konstitutive Promoter ausgewählt aus der Gruppe bestehend aus Promotoren der Glykolysegene, insbesondere pki, gpd oder zwf1, tef1a, act, cox4, neg1 und sar1.

Ein weiterer Aspekt der vorliegenden Erfindung betrifft einen Vektor oder ein Vektor-Set umfassend ein oder die Nukleinsäuremolekül(e) gemäß der vorliegenden Erfindung.

Ein noch weiterer Aspekt der vorliegenden Erfindung betrifft eine Pilzzelle der Gattung Trichoderma umfassend ein oder die Nukleinsäuremolekül(e) oder einen Vektor oder ein Vektor-Set gemäß der vorliegenden Erfindung.

In die erfindungsgemäße Pilzzelle können ein oder mehrere Nukleinsäuremoleküle bzw. Vektoren umfassend Nukleinsäuresequenzen kodierend für N-Acetylglukosamin-2-Epimerase und N-Acetylneuraminsäure-Synthase eingebracht werden. Die für die Enzyme kodierenden Abschnitte sind operativ an einen konstitutiven Promotor gebunden.

Das erfindungsgemäße Nukleinsäuremolekül bzw. der erfindungsgemäße Vektor bzw. das Set werden in die Wirtszelle mit allgemein bekannten Verfahren eingebracht.

Die erfindungsgemäße Pilzzelle gehört der Gattung Trichoderma an. Mitglieder der Gattung Trichoderma sind in der Lage durch Abbau von Chitin N-Acetylglukosamin in einer ausreichenden Menge bereit zu stellen, um hinreichend NeuNAc unter Zuhilfenahme von rekombinant exprimierter N-Acetylglukosamin-2-Epimerase und N-Acetylneuraminsäure-Synthase zu synthetisieren. Daher werden erfindungsgemäß Pilzzellen dieser Gattung besonders bevorzugt eingesetzt.

Wird als Ausgangssubstanz Chitin verwendet, wird dieses in N-Acetyl Glucosamin abgebaut. Dieses Monomer kann sowohl als Kohlenstoff als auch als Stickstoffquelle für das Zellwachstum, sowie als Baustein für die Zellwandbiosynthese (ein wesentlicher Bestandteil ist Chitin) als auch für die Synthese von N-Acetyl Neuraminsäure verwendet werden. Ein induzierbares System setzt punktuell ein und überlastet somit die Verfügbarkeit von N-Acetyl Glucosamin. Im Gegensatz dazu wird durch ein konstitutives System kontinuierlich N-Acetyl Glucosamin abgezogen und somit eine kontinuierliche Produktbildung ermöglicht.

Gemäß einer besonders bevorzugten Ausführungsform der vorliegenden Erfindung ist die Pilzzelle Trichoderma reesei.

Die erfindungsgemäße Pilzzelle umfasst mindestens ein Nukleinsäuremolekül, dessen Nukleinsäuresequenz für eine N-Acetylglukosamin-2-Epimerase und eine N-Acetylneuraminase-Synthase kodiert und operativ an einen in Pilzzellen aktiven konstitutiven Promoter gebunden ist.

Ein weiterer Aspekt der vorliegenden Erfindung betrifft ein Verfahren zur Herstellung von N-Acetylneuraminsäure (NeuNAc) umfassend das Kultivieren von Pilzzellen gemäß der vorliegenden Erfindung in Anwesenheit einer N-Acetyl-D-Glukosamin-Quelle.

Um NeuNAc mit einer N-Acetylglukosamin-2-Epimerase und einer N-Acetylneuraminsäure-Synthase herzustellen, wird N-Acetyl-D-Glukosamin als Substrat benötigt. Daher ist es notwendig Pilzzellen zu verwenden, die in der Lage sind dieses Substrat zur Verfügung zu stellen.

Gemäß einer bevorzugten Ausführungsform der vorliegenden Erfindung ist die N-Acetyl-D-glukosamin-Quelle Chitin.

Die gegenständliche Erfindung wird anhand der folgenden Figuren und Beispiele eingehender illustriert, ohne jedoch auf diese beschränkt zu sein.

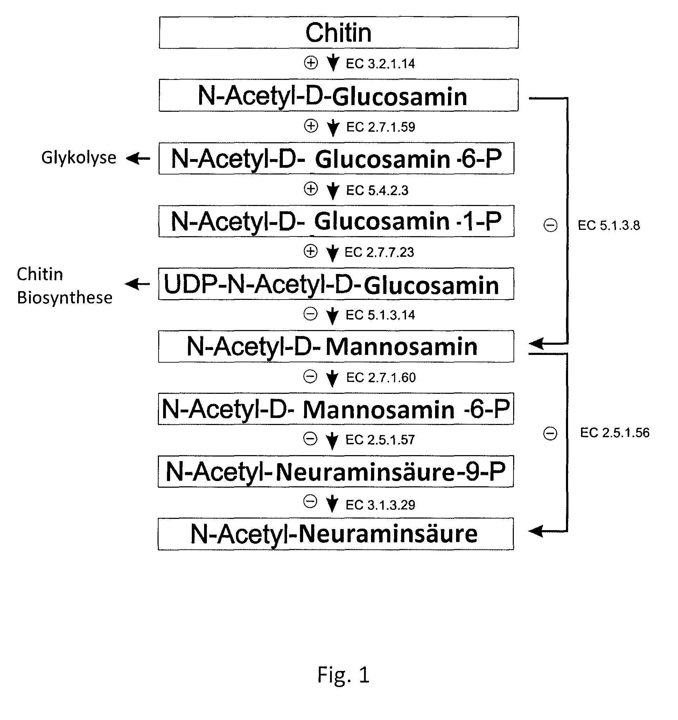

Fig. 1 gibt einen Überblick über die möglichen metabolischen Reaktionswege ausgehend vom Polymer Chitin bis zur Bildung von NeuNAc. Metabolische Zwischenprodukte sind in Rechtecken dargestellt und Pfeile symbolisieren enzymkatalysierte Reaktionen. Neben dem Pfeil ist die korrespondierende EC Nummer der Enzymre-aktion angeführt. Ein eingekreistes Plus bezeichnet eine Enzym-reaktion, die im Genom von *Trichoderma reesei* einem Gen zugewiesen werden konnte (http://genome.jgipsf.org/Trire2/Trire2.home.html). Im Falle von eingekreisten Minus kann kein annotiertes Gen im derzeit publizierten Genom gefunden werden.

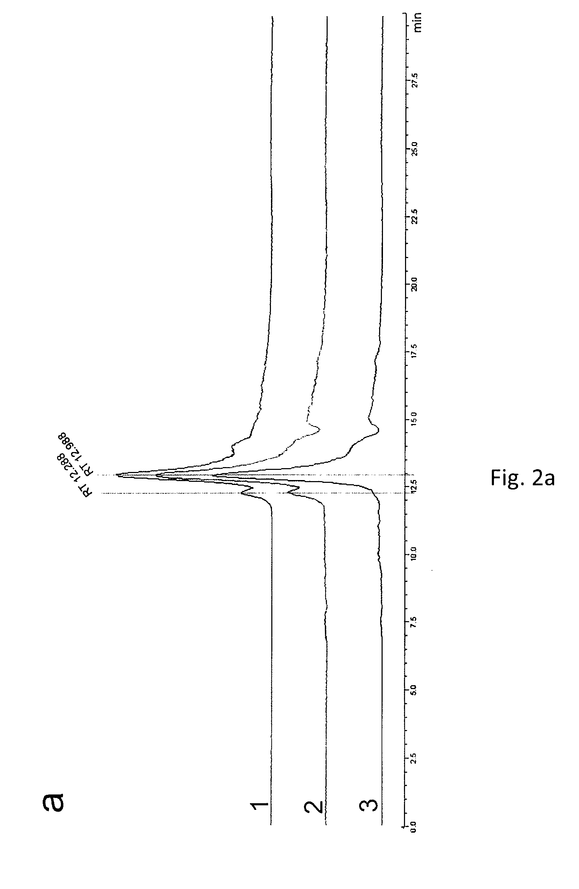

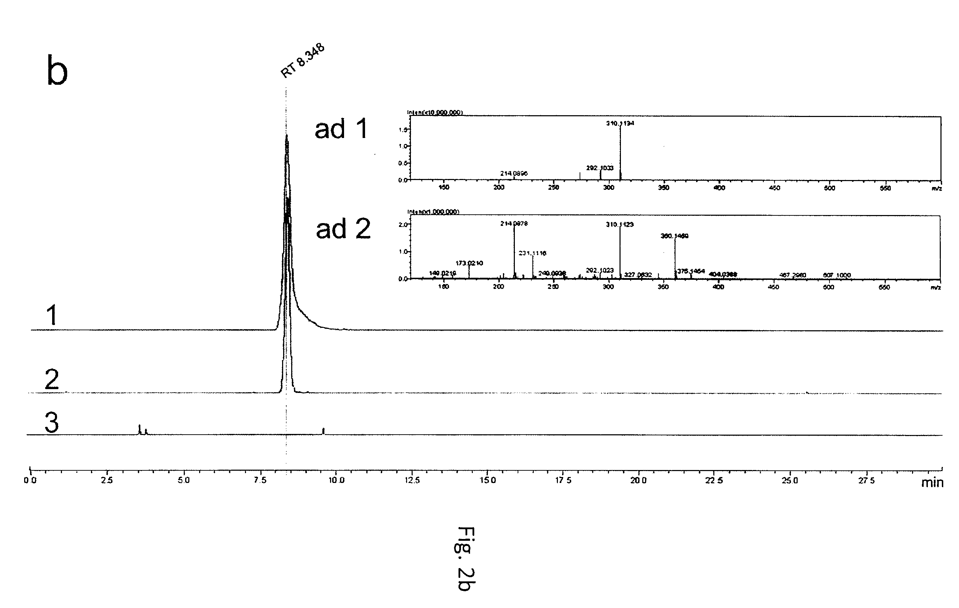

Fig. 2 zeigt die Bildung von NeuNAc in einer in vitro Reaktion mit heterolog expremierten T. reesei Protein im transgenen Stamm PEC/PSC1 unter Verwendung der Substrate GlcNAc, ATP und PEP.
   (a) Die extrahierten Ionenchromatogramme (EIC) einer HPLC-MS-Analyse bei einer Masse von 222.098 atomaren Masseneinheiten (amu) sind dargestellt. Diese Masse korrespondiert mit der Masse des [GlcNAc+H]+ Ions sowie mit der des [ManNAc +H]+ Ions. Die Retentionszeit (RZ) von GlcNAc (12,988 min) sowie ManNAc (12.288) wurden mit reinen Standards beider Substanzen bestimmt und diese ist mit einer vertikalen Linie im Chromatogramm eingezeichnet. (1) Chromatogramm der in vitro Enzymreaktion mit GST-Fusionsproteinen von GlcNAc-2-epimerase und NeuNAc-Synthase, welche in E. coli expremiert wurden. (2) Chromatogramm der Enzymreaktion mit dem zellfreien Extrakt des transgenen PEC/PSC1 Stammes. (3) Chromatogramm der Reaktion mit dem zellfreien Extrakt des Ausgangsstammes QM9414 als Negativkontrolle.
   (b) Die EICs bei einer Masse von 310.1134 amu werden gezeigt, welche mit der Masse des [NeuNAC+H]+ Ions übereinstimmt und bei einer Retentionszeit von 8.345 min detektiert werden kann. Im Falle von Chromatogramm (1), (2) bzw. (3) handelt es sich um dieselben Proben wie unter Punkt (a) geschildert, wobei Chromatogramm (2) gegenüber Chromatogramm (1) um das 10-fache und Chromatogramm (3) um das 1000-fache verstärkt wurden.(ad 1) Beinhaltet das Massenspektrum zu Chromatogramm (1) bei der Retentionszeit von 8.345 min. (ad 2) Zeigt das Massenspektrum von Chromatogramm (2) bei der Retentionszeit von 8.348 min.

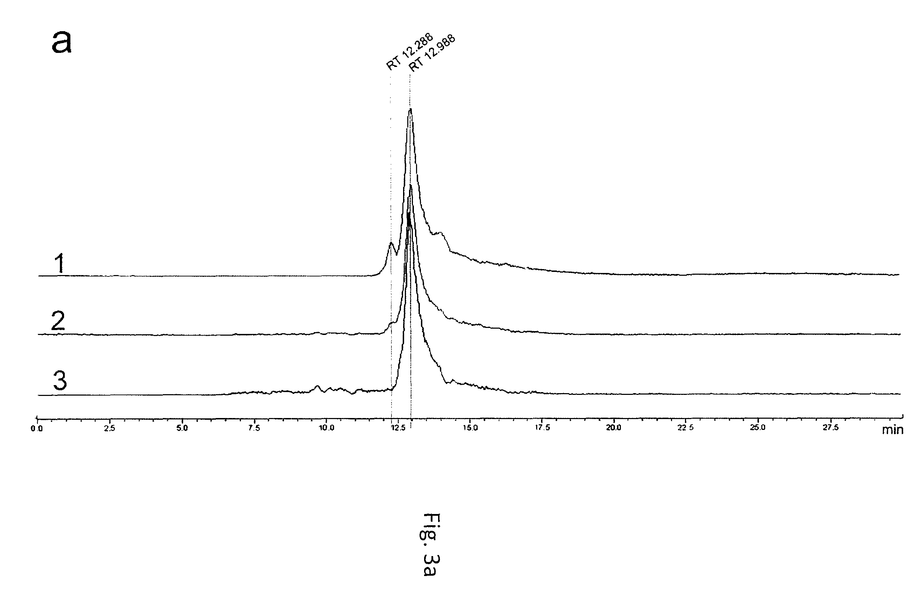

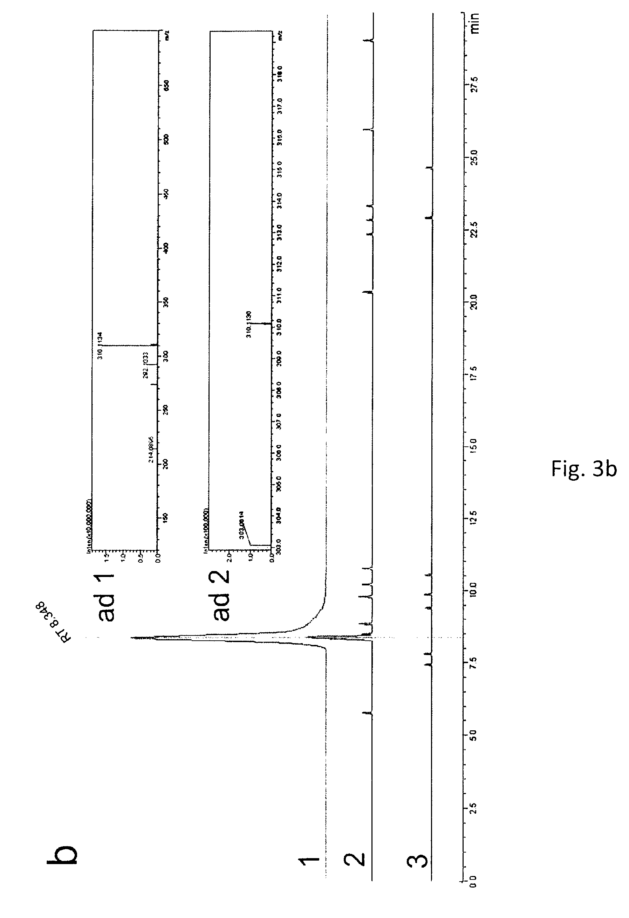

Fig. 3 zeigt die Bildung von NeuNAc in vivo in T. reesei im transgenen Stamm PEC/PSC1 nach Kultivierung auf GlcNAc für 66 h.
   (a) Die EICs der HPLC-MS-Analyse bei einer Masse von 222.097 amu werden gezeigt([GlcNAc+H]+ Ion sowie [ManNAc +H]+ Ion). Die Retentionszeit (RZ) von GlcNAc (12,988 min) sowie ManNAc (12.288) wurden mit reinen Standards beider Substanzen bestimmt und diese ist mit einer vertikalen Linie im Chromatogramm eingezeichnet. (1) Chromatogramm der in vitro Enzymreaktion mit GST Fusionsproteinen von GlcNAc-2-epimerase und NeuNAc Synthase, welche in E. coli expremiert wurden. (2) Chromatogramm des zellfreien Extraktes des transgenen PEC/PSC1 Stammes. (3) Chromatogramm des zellfreien Extraktes des Ausgangsstammes QM9414 als Negativkontrolle.
   (b) Die EICs bei einer Masse von 310.1134 amu werden gezeigt, welche mit der Masse des [NeuNAC+H]+ Ions korrespondiert und bei einer Retentionszeit von 8.345 min detektiert werden kann. Im Falle von Chromatogramm (1), (2) bzw. (3) handelt es sich um dieselben Proben wie unter Punkt (a) geschildert, wobei Chromatogramm (2) gegenüber Chromatogramm (1) um das 100 fache und Chromatogramm (3) um das 1000 fache verstärkt wurden. (ad 1) Beinhaltet das Massenspektrum zu Chromatogramm (1) bei der Retentionszeit von 8.345 min. (ad 2) Zeigt das Massenspektrum von Chromatogramm (2) bei der Retentionszeit von 8.348 min.

### BEISPIEL:

### Materialien und Methoden

### Stämme und Kultivierungsbedingungen

Trichoderma reesei (Hypocrea jecorina) QM9414 (ATCC 26921) wurde als Ausgangsstamm in diesem Beispiel verwendet und wurde auf Malzextraktagar kultiviert.

Myzelien für die in vitro Enzymreaktionen wurden aus Kultivierungen der Stämme in 1000-mL-Erlenmeyerkolben mit je 200 mL 3% (w/v) Malzextraktmedium gewonnen. Die Flaschen wurden mit 10^8 Konidien pro Liter inokuliert und die Kultivierung erfolgte bei 30°C und 250 rpm für 40 h. Die Kultivierung von T. reesei auf kolloidalem Chitin wurde in 1000-mL-Erlenmeyerkolben mit je 200 mL Mandels-Andreotti Medium beinhaltend 1 % (w/v) kolloidales Chitin sowie 0.1 % (w/v) Bacto Pepton durchgeführt. Die Inokulierung erfolgte mit 10^8 Konidien pro Liter und die Inkubation erfolgte auf 30 °C / 250 rpm für 90 h.

Für die in vivo Herstellung von NeuNAc wurden die entsprechenden T. reesei Stämme direkt in 250 mL Mandels-Andreotti Medium mit 1% (w/v) GlcNAc bei 30°C und 250 rpm für 66 h kultiviert (Inokulation mit 10^8 Sporen/Liter).

### Synthetische Gene und Plasmidkonstruktion

Das synthetische Gen tbage wurde anhand der Proteinsequenz von Anabaena sp. CH1 GlcNAc-2-epimerase (GenBank: ABG57042) generiert indem die Proteinsequenz mit der Software GeneOptimizere® (Geneart, Deutschland) in eine DNA-Sequenz übersetzt wurde. Hierbei wurde die DNA-Sequenz hinsichtlich der Kodonverwendung von T. reesei (Tabelle 1) optimiert:

>tbage
tctagaatgggcaagaacctccaggccctggcccagctctacaagaacgccctcctca-
acgacgtcctgcccttctgggagaaccacagcctcgacagcgagggcggctacttcac-
ctgcctcgaccgccagggcaaggtctacgacaccgacaagttcatctggctccagaac-
cgccaggtctggaccttcagcatgctctgcaaccagctggagaagcgcgagaactggctcaa-
gatcgcccgcaacggcgccaagttcctcgcccagcacggccgcgacgacgagggcaactgg-
tactttgccctgacccgcggcggcgagcctctggtccagccctacaacatcttca-
gcgactgcttcgccgccatggccttcagccagtacgccctcgccagcggcgag-
gagtgggccaaggacgtcgccatgcaggcctacaacaacgtcctccgccgcaaggacaac-
cccaagggcaagtacaccaagacctaccccggcacccgccccat-
gaaggccctggctgtccccatgatcctcgccaacctcaccctggagatg-
gagtggctcctcccccaggagaccctggagaacgtcctcgccgccaccgtccaggaggtcat-
gggcgacttcctcgaccaggagcagggcctcatg-
tacgagaacgtcgcccccgacggcagccacatcgactgcttcgagggccgcctcatcaac-
cccggccacggcatcgaggccatgtggttcatcatg-
gacatcgcccgccgcaagaacgacagcaagaccatcaaccaggccgtcgacgtcgtcctca-
acatcctcaacttcgcctgggacaacgagtacggcggcctctactacttcatg-
gacgccgccggccacccccccagcagctggagtgggaccagaagctctggtgggtccacctg-
gagagcctcgtcgccctcgccatgggctaccgcctcaccggccgcgacgcctgctgggcctgg-
tatcagaagatgcacgactacagctggcagcacttcgccgaccctgagtacggcgagtggt-
cggctacctcaaccgcgaggcgaggtcctcctcaacctcaagggcggcaagtg-

gaagggctgcttccacgtcccccgcgccatgtacctctgctggcagcagttcgaggccctca-
gctaatgcat

In analoger Vorgehensweise wurde das synthetische Gen tneub erzeugt, dem die Proteinsequenz der NeuNAc-Synthase von Campylobacter jejuni NCTC11168 zugrundeliegt (http://old.genedb.org/genedb/cjejuni/index.jsp, Cj1141) und dessen DNA-Sequenz ebenfalls an die Kodonverwendung von T. reesei angepasst wurde:
>tneub

tctagaatgcagatcaagatcgacaagctcaccatcagccagaagaacccctcatca-
tccccgagatcggcatcaaccacaacggcagcctggagatcgccaagctcat-
ggtcgacgccgccaagcgagccggcgccaagatcatcaagcaccagacccacatcgtcgag-
gacgagatgagccaggaggccaagaacgtcatccccggcaacgccaacatcagcatctacga-
gatcatggagcagtgcgccctcaactacaaggacgagctggccctcaaggag-
tacgtcgagaagcagggcctcgtctacctcagcacccccttcagccgcgccgccaac-
cgcctggaggacatgggcgtcagcgcctacaagatcggcagcggcgagtgcaacaactac-
cccctgatcaagcacatcgcccagttcaagaagcccatgatcatcagcaccggcat-
gaacagcatcgagagcatcaagcccaccgtcaagatcctccgcgactacgagatccccctt-
cgtcctcctgcacaccaccaacctctacccacccccagccac-
ctcgtccgcctccaggccatgctggagctgtacaaggagttcaactgcctctacggcctca-
gcgaccacacgacgaacaacctcgcctgcatcggcgccatcgccctcggcgccagcgtcctg-
gagcgccacttcaccgacaccatggaccgcaagggccccgacatcgtctgcagcatg-
gacgagagcaccctcaaggacctcatcaaccagacccaggagat-
ggtcctcctccgcggcgacaacaacaagaacccccctgaaggaggagcaggtcacca-
tcgacttcgccttcgccagcgtcgtcagcatcaaggacatcaagaagggcgagatcctca-
gcatggacaacatctgggtcaagcgccccagcaagggcggcatcagcgccaaggactt-
cgaggccatcctcggcaagcgcgccaagaaggacatcaagaacaacatccagctcacctgg-
gacgacttcgagtaatgcat

Für die Konstruktion der Plasmide pMS-PEC und pMS-PSC wurden die synthetischen Gene tbage und tneub aus deren Produktions-plasmid mittels XbaI/NsiI-Restriktionsverdau herausgeschnitten und in das Plasmid pRLMₑₓ₃₀ (Mach, R. L et al. 1994. Curr Genet 25:567-70) eingefügt wobei das hph Gen zwischen der XbaI und NsiI Schnittstelle durch tbage beziehungsweise tneub ersetzt wurde.

Für die Konstruktion von pGEX-epi und pGEX-syn wurde das Plasmid pGEX4T-2 (GE Healthcare, UK) mit EcoRI und XhoI verdaut. Eine doppelsträngige DNA, bestehend aus den oligomeren Nukleotiden GEXfw und GEXrev (Tabelle 1), wurde in den offenen pGEX4T-2 eingefügt wodurch das Plasmid pGEX-MS entstand und die neuen Schnittstellen XbaI und NsiI generiert wurden. tbage und tneub wurden in pGEX-MS über die Schnittstellen XbaI/NsiI eingebracht woraufhin die Plasmide pGEX-epi sowie pGEX-syn entstanden.

**Tabelle 2: Nukleotidsequenzen der verwendeten Oligomere**

| **Name** | **Sequenz (5'→3')** | **Verwendung** |
|---|---|---|
| NANASfw | GTGGTGTGCAGGAGGACGAA | qPCR tneub |
| NANASrev | CAAGCACATCGCCCAGTTCAAG | qPCR tneub |
| ManEfw | GCGATCTTGAGCCAGTTCTC | qPCR tbage |
| ManErev | GCTACTTCACCTGCCTCGAC | qPCR tbage |
| GEX-MSfw | AATTCCTTCTAGAGATATGCATC | Konstruktion von pGEX-MS |
| GEX-MSrev | TCGAGATGCATATCTCTAGAAGG | Konstruktion von pGEX-MS |
| pkifw R | CTGCGACACTCAGAACATGTACGT | qPCR pki cDNA |
| pkifw D | GCTCTGCTTGGAACCTGATTGA | qPCR pki DNA |
| pkirev | GGTCTGGTCGTCCTTGATGCT | qPCR pki |
| sar1fw | TGGATCGTCAACTGGTTCTACGA | qPCR sar1 |
| sarlrev | GCATGTGTAGCAACGTGGTCTTT | qPCR sar1 |

### Protoplastentransformation von T. reesei

Die Protoplastentransformation von T. reesei wurde wie in einem früheren Artikel (Gruber, F., et al., 1990. Curr Genet. 18, 71-6) erwähnt ausgeführt. Eine Gesamtmenge von 10 µg DNA wurde pro Transformation verwendet. In einer Kotransformation wurde pMS-PEC (4 µg) und pMS-PSC (4 µg) gemeinsam mit dem Plasmid pHylox2 (2 µg), welches eine Hygromycin B Resistenz vermittelt, transformiert. Rekombinante Stämme wurden auf Hygromycin B Resistenz selektiert.

### RNA Analyse

RNA Extraktion, reverse Trankription und qPCR wurden wie in einem früheren Artikel beschrieben ausgeführt. Oligomere Nukleotidsequenzen welche als Primer eingesetzt wurden sind in der Tabelle 1 angeführt. Sar1 wurde als Referenzgen für die Normalisierung der RT-qPCR verwendet. Für das Gen tbage wurden in der qPCR die Primer ManEfw und ManErev bei einer optimalen Elongationstemperatur von 64°C und 2 mM MgCl2 verwendet. Für das Gen tneub wurden in der qPCR die Primer NANAfw und NANArev bei einer optimalen Elongationstemperatur von 64°C verwendet. Für das Gen pki wurden in der qPCR die Primer pkifwR und pkirev bei einer optimalen Elongationstemperatur von 64°C verwendet. Die Datenanalyse wurde mit REST 2008 durchgeführt.

### DNA Analyse

Genomische DNA wurde aus den Pilzmyzel wie in einem früheren Artikel beschrieben isoliert. Die Hybridisierung und Detektion wurde gemäß den Standardarbeitsvorschriften mit dem DIG High Prime DNA Labeling and Detection Starter Kit II (Roche, Schweiz) ausgeführt. qPCR von genomischer DNA wurde mit ∼50 ng genomischer Templat-DNA durchgeführt. Dieselben Primer wie in der RNA Analyse wurden für die Gene tbage und tneub verwendet. Als Referenzgen diente pki, welches mit den Primern pkifwD und pkirev bei einer Elongationstemperatur von 64°C amplifiziert wurde.

### Gluthation S-Transferase (GST) Fusionsproteine

GST-Fusionsproteine von GlcNAc-2-Epimerase (GST:epi) sowie NeuNAc-Santhase (GST:syn) wurden durch Expression der Plasmide pGEX-epi und pGEX-syn in *E*. *coli* BL21(DE3) Zellen erzeugt. Die Reinigung der Fusionsproteine wurde mithilfe von GSTrap™FF Säulchen 1 mL Säulenvolumen (GE Healthcare), unter Verwendung des Standardarbeitsprotokolls realisiert.

### Enzymreaktion mit zellfreien Extrakten

Geerntetes Myzel wurde in flüssigem Stickstoff zu einem feinen Puffer zerrieben und sodann in einem 0.1 M Bicine Puffer (pH 8), der Proteaseinhibitoren (2 µM leupeptin, 1 µM pepstatin A, 10 µM PMSF) beinhaltet, resuspendiert (0.3 g Myzelpulver/1 mL Puffer). Die Suspension wurde mit einem Ultraschallstab Sonifier® 250 Cell Disruptor (Branson, US) (Einstellungen: Power 40%, duty cycle 50%, 20 s Aktion, 40 s Pause, 10 Zyklen) weiter aufgeschlossen und unlösliche Bestandteile durch Zentrifugation (10 min, 13000 x g, 4 °C) abgetrennt. Der Überstand wurde in einem für die enzymatische Reaktion herangezogen. Die Enzymreaktion wurde ähnlich ausgeführt wie von Vann et al. beschrieben (Vann, W. F., et al. 1997. Glycobiology 7:697-701). Die Reaktion um die Aktivität von GlcNAc-2-Epimerase nachzuweisen beinhaltet 10 mM GlcNAc, 0.2 mM ATP, 100 mM Bicine Puffer (pH 8) und 10-40 µL zellfreien Extrakt in einem Gesamtvolumen von 100 µL. Die reaktion um die Aktivität von NeuNAc-Synthase nachzuweisen beinhaltet 10 mM ManNAc, 10 mM PEP, 12.5 mM MnC12, 100 mM Bicine Puffer (pH 8) und 10-40 µL zellfreien Extrakt in einem Gesamtvolumen von 100 µL. Die Kombinationsrektion um sowohl die Aktivität von GlcNAc-2-Epimerase als auch NeuNAc-Synthase nachzuweisen beinhaltet 10 mM GlcNAc, 10 mM PEP, 12.5 mM MnCl2, 100 mM Bicine Puffer (pH 8) und 40 µL zellfreien Extrakt in einem Gesamtvolumen von 100 µL. Alle Reaktionen wurden für 60 min bei 37 °C inkubiert, durch Erhitzen auf 85 °C für 10 min inaktiviert und dann mittels HPLC analysiert. Als Positivkontrolle wurden je 5 µL (1 µg/ µL) der GST-Fusionsproteine GST:epi und GST:syn in der Enzymreaktion anstelle der zellfreien Extrakte verwendet.

### Chitinase Enzymreaktion

Die Freisetzung von GlcNAc aus dem Polymer Chitin wird in dieser Reaktion gemessen. Das Chitin wurde sowohl als Rohchitin aus Krabbenpanzern sowie als kolloidales Chitin in einem 30 mM Phosphatbuffer (pH 4.7) eingesetzt. 5, 10 oder 50 µL von Kulturüberstand wurden in einem Totalvolumen von 1.5 mL gemessen. Die Raktion wurde bei 37 °C für 20 h inkubiert und dann durch Erhitzen bei 90°C für 10 min inaktiviert. Die Bildung von GlcNAc wurde auf der HPLC gemessen.

### NeuNAc Detektion in zellfreien Extrakten

Geerntetes Myzel von T. reesei wurde in flüssigem Stickstoff zu einem feinen Pulver zerrieben und in bidestillierten Wasser resuspendiert (0.3 g Myzelpulver / 1 mL Wasser). Die Suspension wurde mit einem Ultraschallstab Sonifier® 250 Cell Disruptor (Branson, US) (Einstellungen: Power 40%, duty cycle 50%, 20 s Aktion, 40 s Pause, 10 Zyklen) weiter aufgeschlossen und unlösliche Bestandteile durch Zentrifugation (10 min, 13000 x g, 4 °C) abgetrennt. Der Überstand wurde durch einen 0.45 µm Filter filtriert und mittels HPLC-MS analysiert.

### HPLC-MS Analyse

Die Bildung von NeuNAc und ManNAc in der Enzymreaktion sowie im zellfreien Extrakt wurde auf einer HPLC-MS (IT-TOF-MS) (Shimadzu, Japan) mit einer Rezex^{™} RHM Monosaccharide H⁺-Säule (8%, 300 x 7.8 mm) (Phenomenex, USA) gemessen. Die mobile Phase bestand aus Wasser mit 0.1% (v/v) Trifluoressigsäure und der Fluss wurde auf 0.6 mL / min gesetzt. Die Säulentemperatur betrug 80°C und 10 µL Probe wurde auf die Säule aufgegeben. Die Detektion erfolgte im ESI⁺ Modus und es wurde ein Scanbereich von 60-600 amu abgedeckt.

### Ergebnisse

### In silico Analyse eines NeuNAc Biosyntheseweges in T. reesei

Derzeit gibt es in der aktuellen Literatur keine Hinweise darauf, dass NeuNAc in Trichoderma reesei natürlich gebildet werden kann. Daher wurden in silico die bekannten metabolischen Reaktionen überprüft, die zur Bildung von NeuNAc führen und ob diese in T. reesei vorkommen. Fig. 1 zeigt die derzeit bekannten enzymkatalysierten Prozesse auf, die zur Bildung von NeuNAc führen und das Biopolymer Chitin als Ausgangssubstanz verwenden. In Trichoderma finden sich Enzyme, die für die Katabolisierung von Chitin notwendig sind. Der erste Schritt von Chitin zum Monomer GlcNAc wird hierbei von Chitinasen (3.2.1.14) katalysiert. Weiters ist für die Katabolisierung von Chitin die Aktivität einer Hexokinase (EC 2.7.1.1), einer GlcNAc-6-Phosphat Deazetylase (EC 3.5.1.25) und einer Glucosamin-6-Phosphat Deaminase (EC 3.5.99.6) notwendig, damit schlussendlich Fruktose-6-Phosphat in die Glykolyse eintreten kann. Für diese Enzymaktivitäten kann in T. reesei zumindest je ein potentielles Enzym im annotierten Genom gefunden werden (Tabelle 3). Zudem können Gene, die für die Biosynthese von Chitin verantwortlich sind gefunden werden, einschließlich einer Phosphoazetylglukosamin Mutase (EC 5.4.2.3), einer UDP-N-GlcNAc Diphosphorylase (EC 2.7.7.23) und mehrerer Chitinsynthasen (EC 2.4.1.16). Es sind aber keine Gene im Genom von T. ressei annotiert, die für die Synthese von ManNAc (EC 5.1.3.8 in Bakterien, EC 5.1.3.4 in Säugetieren) noch für die Synthese von NeuNAc (EC 2.5.1.6. in Bakterien, EC 2.7.1.60, EC 2.5.1.57, EC 3.1.3.29 in Säugetieren) verantwortlich sind.

**Tabelle 3: Genkandidaten für die metabolischen Umsetzungen von Chitin und GlcNAc, die im Genom von T. reesei annotiert sind.**

| **EC Nummer** | **Name** | **Protein Identität** |
|---|---|---|
| EC 3.2.1.14 | Chitinase | 2735, 43873, 53949, 62645, 62704, 66041, 68347, 72339, 80833, 81598, 104401, 110317, 119859, 123354, 124043 |
| EC 2.7.1.1 | Hexokinase | 56129, 73665, 79677 |
| EC 3.5.1.25 | GlcNAc-6-phosphate Deazetylase | 79671 |
| EC 3.5.99.6 | Glucosamin-6-Phosphat Deaminase | 49898 |
| EC 5.4.2.3 | Phosphoazetylglucosaminmutase | 80994 |
| EC 2.7.7.23 | UDP-N-GlcNAc Diphosphorylase | 79568 |
| EC 2.4.1.16 | Chitinsynthase | 51492, 55341, 58188, 71563, 112271, 122172 |

### Ein Gencluster für die katabolische Umsetzung von GlcNAc in Trichoderma reesei

Während der in silico Analyse der Abbauwege für GlcNAc, konnten 3 Genkandidaten (estExt_GeneWisePlus.C_140427, es-tExt_GeneWisePlus.C_140421, estExt_Genewise1.C_140432) kodierend für eine Hexokinase, eine GlcNAc-6-phosphate Deazetylase und eine Glukosamin-6-phosphate Deaminase gefunden werden, die alle in direkter Nachbarschaft im Genom von T. reesei zu finden sind (Lage im Genom auf "scaffold 14": 714385-729984). Ähnliche Cluster kommen auch in anderen filamentösen Pilzen wie Neurospora crassa oder Aspergillus nidulans vor, was auf einen konservierten Cluster für die Katabolisierung von GlcNAc hinweist.

Die Hexokinase (Protein ID 79677), die im Genom von T. reesei annotiert ist, kann daher weiters als GlcNAc Kinase (EC 2.7.1.59) spezifiziert werden, analog der Annotation und Charakterisierung wie sie in Candida albicans zu finden ist (39). Weiters kann das Gen (estExt_GeneWisePlus.C_140419), welches neben der GlcNAc-6-Phosphat Deazetylase (estExt_GeneWisePlus.C_140421) liegt, ebenfalls zu dem Cluster gehören, da ein Homolog dieses Genes in Neurospora crassa als β-N-Azetylglukosaminidase (N. crassa OR74A (NC10): Supercontig 6: 560844-564980) annotiert ist.

### Konstruktion von Expressionsvektoren

Es wurde eine zwei Enzymstrategie für die Produktion von NeuNAc in Trichoderma gewählt, wobei der erste Enzymschritt von einer GlcNAc 2-epimerase (EC 5.1.3.8) und der zweite von einer NeuNAc Synthase (EC 2.5.1.99) katalysiert wird. Als Kandidaten wurde die Proteinsequenz von der GlcNAc 2-Epimerase aus Anabaena sp. CH1 (GenBank: ABG57042) und für die NeuNAc Synthase die Proteinsequenz aus Campylobacter jejuni NCTC11168 (Cj1141) gewählt. Die Proteinsequenzen wurden in DNA Sequenzen mithilfe der Software GeneOptimizer® (Geneart) übersetzt und die Kodonverwendung an die von T. reesei (Tabelle 1) angepasst. Die resultierenden synthetischen Gene wurden tbage und tneub genannt.

Die kodierenden Sequenzen wurden in das Plasmid pRLMex30 eingesetzt, wobei die kodierende Sequenz für das hph Gen aus diesem Plasmid ersetzt wurde. Somit standen beiden Gene unter der Kontrolle des konstitutiven pki Promotors und des cbh2 Terminators (Plasmide pMS-PEC mit tbage sowie pMS-PSC mit tneub).

Um beide Gene auch unter einem induzierbaren System exprimieren zu können, wurde der pki Promotor durch den xyn1 Promotor ersetzt (Plasmide pMS-XEX mit tbage, sowie pMS-XSC mit tneub).

**Tabelle 4: Vergleich eines induzierbaren Promotorsystems (xyn1) gegenüber eines konstitutiven Promotorsystems (pki) (n.b. nicht bestimmt, + vorhanden, - nicht vorhanden)**

| | **Promotor** | **DNA im Genom** | | **Transkriptbildung** | | **Enzymaktivität** | |
|---|---|---|---|---|---|---|---|
| Stamm | | Epimerase | Synthase | Epimerase | Synthase | Epimerase | Synthase |
| XEX5 | xyn1 | + | n.b. | + | n.b. | - | n.b. |
| XEX11 | xyn1 | + | n.b. | + | n.b. | - | n.b. |
| XSC3 | xyn1 | n.b. | + | n.b. | + | n.b. | - |
| XSC13 | xyn1 | n.b. | + | n.b. | + | n.b. | - |
| PEC11 | pki | + | n.b. | + | n.b. | + | n.b. |
| PEC15 | pki | + | n.b. | + | n.b. | + | n.b. |
| PEC17 | pki | + | n.b. | + | n.b. | + | n.b. |
| PSC15 | pki | n.b. | + | n.b. | + | n.b. | + |
| PSC16 | pki | n.b. | + | n.b. | + | n.b. | + |
| PSC17 | pki | n.b. | + | n.b. | + | n.b. | - |
| XEX/XSC1 | xyn1 | + | + | + | + | - | - |
| XEX/XSC5 | xyn1 | + | + | + | + | - | - |
| PEC/PSC1 | pki | + | + | + | + | + | + |
| PEC/PSC10 | pki | + | + | + | + | + | - |

Um Trichoderma reesei Stämme zu erzeugen, die die Fähigkeit haben NeuNAc herzustellen, wurde der Ausgangsstamm QM9414 mit verschiedenen Kombinationen der Plasmide pMS-PEC, pMS-PSC, pMS-XEX sowie pMS-XSC und pMS-Hylox2 (beinhaltet den Selektionsmarker hph zwischen zwei loxP Sequenzen) transformiert. Es wurden die Plasmide beinhaltend die Gene tbage und tneub sowohl einzeln als auch in Kombination tneub/tbage transformiert.

Ausgewählte Transformanten wurden hinsichtlich der Integration der transformierten DNA ins Genom sowie ihrer Transkriptbildung und Enzymaktiviät von GlcNAc 2-Epimerase und NeuNAc Synthase untersucht. Die Ergebnisse sind in Tabelle 4 dargestellt. Es ist ersichtlich, dass mit dem xyn1 Promoter zwar eine Transkriptbildung nachweisbar ist, aber keine Enzymaktivität der beiden heterolog exprimierten Enzyme nachweisbar war. Es wurde daher nur mit den Stämmen, die die beiden Gene unter der Kontrolle des pki Promoters expremierten, weitergearbeitet.

Die beiden Stämme PEC/PSC1 und PEC/PSC10 wurden weiterführend auf deren genomisches Kopienzahlenverhältnis von tbage und tneub untersucht. Tabelle 5 zeigt die Ergebnisse dieser Untersuchung.

**Tabelle 5: Vergleich der Gentranskription und Genkopienanzahl zwischen 2 transgenen T. reesei Stämmen**

| **Genbezeichnung** | **Transkriptverhältnis PEC/PSC1 / PEC/PSC10 Median [95% K.I.]** | **Kopienverhältnis PEC/PSC1 / PEC/PSC10 Median [95% K.I.]** |
|---|---|---|
| tbage | 2.021 [1.589-2.836] | 1.810 [1.376-2.585] |
| tneub | 0.479 [0.385-0.622] | 0.400 [0.320-0.492] |

Der PEC/PSC1 Stamm zeigt eine ungefähr 2 fach höhere Transkription des tbage Gens als der PEC/PSC10 Stamm. Im Gegensatz dazu verfügt der PEC/PSC10 Stamm über eine ungefähr 2 fach höhere Transkription des tneub Gens als der Stamm PEC/PSC1. Diese unterschiedlichen Transkriptniveaus können durch die unterschiedlichen Kopienzahlen der beiden Gene im Genom der zwei Stämme erklärt werden. Das Verhältnis der Kopienzahl in den beiden Stämmen wurde mittels qPCR von genomischer DNA gemessen, wobei als Referenz das Gen kodierend für die Pyruvatkinase (pki) diente. Die Kopienverhältnisse in den beiden Stämmen verhielten sich dabei wie die Transkriptverhältnisse, womit die unterschiedlichen Transkriptverhältnisse durch das Kopienzahlenverhältnis erklärbar sind und jede Kopie des Genes scheinbar mit der gleichen Effizienz transkribiert wird (Tabelle 5).

### Heterologe Proteinexpression von GlcNAc 2-Eppimerase und NeuNAc Synthase in Trichoderma reese

Nach der Kultivierung des Trichoderma reesei Stammes wurde der zellfreie Extrakt auf die Gegenwart von GlcNac 2-Epimerase und NeuNAc Synthase getestet. Die Umsetzung der Substrate PEP und GlcNAc hinzu ManNAc und NeuNAc wurde nach Zugabe des enzym- haltigen zellfreien Extraktes gemessen. Die Umsetzung wurde mittels HPLC-MS analysiert und die entsprechenden Chromatogramme sind in Abbildung 2 dargestellt. Als Positivkontrolle wurden GST-Fusionsproteine von GlcNAc 2-Epimerase (tbage) und NeuNAc Synthase (tneub), welche durch Expression in E. coli erzeugt wurden, in der Umsetzung eingesetzt.

Die Bildung von ManNAc und NeuNAc zeigt, dass die beiden synthetischen Gene tbage und tneub funktionell in Trichoderma expremiert werden (Fig. 2a2 und Fig. 2b2). Ebenso zeigt die Positivkontrolle mit den GST Fusionsproteinen die Bildung von ManNAc und NeuNAc (Fig. 2a1 und Fig. 2b1). Aber weder ManNAc noch NeuNAc werden in der Enzymreaktion gebildet, wenn ein Extrakt aus dem Ausgangsstamm QM9414 eingesetzt wird. Dieses Ergebnis zeigt, dass keine nennenswerte GlcNAc 2-Epimerase Aktivität im Ausgangsstamm vorhanden ist. Zudem wurde auch auf die reine NeuNAc Synthase Aktivität im Stamm QM9414 getestet, indem ManNAc und PEP als Substrat in der Enzymreaktion eingesetzt wurden. Aber auch in diesem Fall konnte keine Aktivität im Ausgangsstamm festgestellt werden, was die Vermutung nahelegt, dass keine NeuNAc Synthase noch GlcNAc 2-Epimerase Aktivität in natürlichen Isolaten von Trichoderma reesei besteht.

### Wachstum von Trichoderma reesei auf kolloidalem Chitin und Freisetzung von GlcNAc

Um die Hydrolyse von Chitin in das Monomer GlcNAc zu untersuchen, kultivierten wir T. reesei PEC/PSC1 auf kolloidalem Chitin als Kohlenstoffquelle. Während der Anzucht wurde der Anstieg der Chitinaseaktivität verfolgt. Nach 90 h Anzuchtdauer erreichte die Chitinaseaktivität ein Maximum und der Überstand wurde auf die Fähigkeit zur Hydrolyse von Chitin getestet. Tabelle 6 präsentiert die Ergebnisse. Zehnmal mehr GlcNAc kann von kolloidalem Krabbenpanzerchitin freigesetzt werden als von unbehandeltem Krabbenpanzerchitin. Freigesetztes GlcNAc kann als Ausgangssubstanz für die Produktion von NeuNAc mit dem Stamm PEC/PSC1 genutzt werden.

**Tabelle 6: Chitinaseaktiviät gebildet durch Kultivierung von T. reesei PEC/PSC1 Stamm auf 1% Chitin.**

| **Substrat** | **Chitinase Aktivität ^{a} [mU/mL]** |
|---|---|
| crab-shell chitin | 2,7 ± 0,5 |
| colloidal crab-shell chitin | 25,0 ± 0,9 |

| | |
|---|---|
| ^{a} 1U: Freisetzung von 1µmol GlcNac/min bei 37 °C | |

### In vivo Bildung von NeuNAc in T. reesei

Im folgenden Experiment soll geklärt werden, ob die 2 heterolog expremierten Enzyme auch in vivo funktionstüchtig sind und die Fähigkeit besitzen NeuNAc aus deren Nährsubstrat zu bilden. Dafür wurde der rekombinante Stamm PEC/PSC1 auf GlcNAc kultiviert. Als Negativkontrolle wurde der Ausgangsstamm QM9414 kultiviert. Von beiden Stämmen wurde das Myzel geerntet und auf die Anwesenheit von NeuNAc mittels HPLC-MS untersucht. Die Ergebnisse sind in Abbildung 3 dargestellt.

Der rekombinante Stamm PEC/PSC1 bildet ManNAc (Abbildung 3a2) und NeuNAc (Abbildung 3b2, 10 µg pro g Trockenbiomasse). Dieses Ergebnis demonstriert, dass NeuNAc in T. reesei durch Koexpression zweier bakterielle Enzyme erzeugt werden kann. Der Ausgangsstamm QM9414 zeigt keine Bildung von NeuNAc noch von ManNAc (Fig. 3a3 and Fig. 3b3).

### Zusammenfassung der Ergebnisse

In diesem Beispiel wurde die Einbringung eines intrazellulären Syntheseweges zur Herstellung von NeuNAc im Pilz Trichoderma reesei demonstriert. Nach bestem Wissen und Gewissen, ist das die erste Arbeit in der eine intrazelluläre zweistufige Enzym-kaskade in einen filamentösen Pilz eingebracht wird um eine Feinchemikalie wie NeuNAc zu erzeugen. T. reesei ist selbst nicht in der Lage NeuNAc zu bilden, aber der wichtige intermediäre Metabolit GlcNAc wird von T. reesei gebildet. Diese Substanz wird bei der Depolymerisierung des erneuerbaren Rohstoffs Chitin freigesetzt (Tabelle 6). Aufgrund seiner saprophytischen Lebensweise, erzeugt T. reesei eine Vielzahl von Chitinasen (Tabelle 3) und kann das Polymer Chitin effektiv in sein Monomer GlcNAc zersetzen. Die spezifische Biosynthese von NeuNAc beginnt von Intermediaten des Chitin Stoffwechselwegs (GlcNAc bzw. UDP-GlcNAc) (vgl. Fig. 1), welche in T. reesei zur Verfügung stehen. Es können aber keine Gene in diesem Organismus gefunden werden, die eine Ähnlichkeit mit Genen kodierend für eine UDP-GlcNAc-2-Epimerase, eine ManNAc Kinase, eine NeuNAc-9-Phosphat Synthase noch eine NeuNAc-9-phosphatase besitzen. Ein alternativer Syntheseweg für NeuNAc, wie er in Bakterien vorkommt, benötigt die Aktivität einer GlcNAc-2-Epimerase und einer NeuNAc-Synthase (Fig. 1). Auch für diesen Weg existieren keine Gene in Trichoderma reeesei.

In Aspergillus fumigates ist die Anwesenheit von NeuNAc auf der Oberfläche von Konidien nachgewiesen worden, aber auf den Konidien des Trichoderma reesei Stammes QM9414 konnte keine NeuNAc gefunden werden. Weder die nötige Enzymaktivität noch Spuren von ManNAc und NeuNAc konnten in diesem Stamm gemessen werden. Dies zeigt, dass Trichoderma reesei Stämme natürlicherweise weder NeuNAc noch ManNAc erzeugen können. Daher ist es notwendig die entsprechenden Enzymaktivitäten mittels heterologer Expression in diesem Organismus zu erzeugen um NeuNAc zu produzieren. Das erste Enzym in der Kaskade, eine GlcNAc 2-Epimerase, wurde aus Anabaena sp. genommen. Dieses Enzym ist gut charakterisiert und benötigt eine vergleichsweise geringe Menge des Kofaktors ATP (20µM) um seine maximale Aktivität zu entfalten.

Das zweite Enzym, eine NeuNAc Synthase, wurde aus C. jejuni gewählt. Die Kodonverwendung beider Gene wurde auf die Kodonverwendung von Trichoderma reesei optimiert um die Expression der bakteriellen Gene im Pilzwirt zu verbessern. Für die Expression der Gene wurde einerseits der konstitutive Promoter des pki Gens, sowie der gut regulierbare Promoter des xyn1 Gens gewählt. Unter der Kontrolle des xyn1 Promoters konnte keine erfolgreiche Expression der beiden Gene erzielt werden. Zwar konnte gezeigt werden, dass die Gene transkribiert werden, aber keine Enzymaktivität konnte nachgewiesen werden. Unter der Kontrolle des pki Promoters können die beiden heterolog expremierten Gene aber nicht nur transkribiert werden, sondern es konnte auch die korrespondierende Enzymaktivität nachgewiesen werden. In einem Stamm, der beide Gene unter dem konstitutiven pki Promoters expremiert, konnte die Bildung von NeuNAc auch in vivo gezeigt werden. Dazu wurde der Pilz auf dem Biopolymer Chitin kultiviert, was zur Freisetzung des Monomers GlcNAc führte. Bei Kultivierung des rekombinanten Stammes konnte die Bildung von NeuNAc im Myzel nachgewiesen werden (Fig. 3b2).

Durch Einbringung einer zweistufigen Enzymkaskade in Trichoderma reesei wurde gezeigt, dass der Pilz die Fähigkeit erlangt hat NeuNAc zu erzeugen. Dieses Beispiel zeigt auf, dass hochwertige Feinchemikalien aus einem erneuerbaren Rohstoff, wie hier aus Chitin, erzeugt werden können. Aber nicht nur Chitin sondern eine Vielzahl von anderen Kohlenstoffquellen, wie zum Beispiel Zellulose und Hemizellulosen, können von dem saprophytischen Pilz Trichoderma reesei genutzt werden und unterstreichen dessen Potential als Zellfabrik für die Herstellung von verschiedenen Chemikalien eingesetzt zu werden.

## Patentansprüche

1. Isoliertes a) Nukleinsäuremolekül umfassend mindestens einen in Pilzzellen der Gattung Trichoderma aktiven Promoter an dem jeweils eine Nukleinsäuresequenz kodierend für eine N-Acetylglukosamin-2-Epimerase (EC 5.1.3.8) und eine N-Acetylneuraminsäure-Synthase (EC 2.5.1.56) operativ gebunden ist, oder b) Set mit zwei Nukleinsäuremolekülen, wobei ein Molekül für die N-Acetylglukosamin-2-Epimerase (EC 5.1.3.8) und ein Molekül für die N-Acetylneuraminsäure-Synthase (EC 2.5.1.56) kodiert, jeweils umfassend mindestens einen in Pilzzellen der Gattung Trichoderma aktiven Promoter operativ an die N-Acetylglukosamin-2-Epimerase oder N-Acetylneuraminsäure-Synthase kodierende Sequenz gebunden;
wobei gemäß a) oder b) der mindestens eine in Pilzzellen aktive Promoter ein konstitutiver Promoter ist.

2. Nukleinsäuremolekül oder Set gemäß Anspruch 1, **dadurch gekennzeichnet, dass** die Pilzzellen der Gattung Trichoderma Trichoderma reesei Zellen sind.

3. Nukleinsäuremolekül oder Set gemäß Anspruch 1 oder 2, **dadurch gekennzeichnet, dass** der konstitutive Promoter ausgewählt ist aus der Gruppe bestehend aus Promotoren der Glykolysegene, insbesondere pki, gpd oder zwf1, tef1a, act, cox4, neg1 und sar1.

4. Vektor umfassend ein Nukleinsäuremolekül a) gemäß einem der Ansprüche 1 bis 3 oder Vektor-Set umfassend die Nukleinsäuremoleküle b) gemäß einem der Ansprüche 1 bis 3.

5. Pilzzelle der Gattung Trichoderma umfassend ein Nukleinsäuremolekül a) oder die Nukleinsäuremoleküle b) gemäß einem der Ansprüche 1 bis 3 oder einen Vektor oder ein Vektor-Set gemäß Anspruch 4.

6. Pilzzelle gemäß Anspruch 5, **dadurch gekennzeichnet, dass** die Pilzzelle der Gattung Trichoderma Trichoderma reesei ist.

7. Pilzzelle gemäß Anspruch 5 oder 6, **dadurch gekennzeichnet, dass** diese mindestens ein Nukleinsäuremolekül umfasst dessen Nukleinsäuresequenz für eine N-Acetylglukosamin-2-Epimerase und eine N-Acetylneuraminase-Synthase codiert und operativ an einen in Pilzzellen aktiven konstitutiven Promoter gebunden ist.

8. Verfahren zur Herstellung von N-Acetylneuraminsäure (NeuNAc) umfassend das Kultivieren von Pilzzellen der Gattung Trichoderma gemäß einem der Ansprüche 5 bis 7 in Anwesenheit einer N-Acetyl-D-glukosamin-Quelle.

9. Verfahren gemäß Anspruch 8, **dadurch gekennzeichnet, dass** die N-Acetyl-D-glukosamin-Quelle Chitin ist.

## Claims

1. An isolated a) nucleic acid molecule comprising at least one promoter that is active in fungal cells of the genus *Trichoderma* and has a nucleic acid sequence encoding an N-acetylglucosamine 2-epimerase (EC 5.1.3.8) and an N-acetylneuraminic acid synthase (EC 2.5.1.56) operably linked thereto, or b) a set of two nucleic acid molecules, wherein one molecule encodes the N-acetylglucosamine 2-epimerase (EC 5.1.3.8) and one molecule encodes the N-acetylneuraminic acid synthase (EC 2.5.1.56), each of said molecules comprising at least one promoter that is active in fungal cells of the genus *Trichoderma* operably linked to the sequence encoding N-acetylglucosamine 2-epimerase or N-acetylneuraminic acid synthase,
wherein according to a) or b) said at least one promoter that is active in fungal cells is a constitutive promoter.

2. Nucleic acid molecule or set according to claim 1, **characterized in that** the fungal cells of the genus *Trichoderma* are *Trichoderma reesei* cells.

3. Nucleic acid molecule or set according to claim 1 or 2, **characterized in that** the constitutive promoter is selected from the group consisting of promoters of the glycolytic genes, especially of pki, gpd or zwf1, tef1a, act, cox4, neg1 and sar1.

4. A vector comprising a nucleic acid molecule a) according to any one of claims 1 to 3, or set of vectors comprising the nucleic acid molecules b) according to any one of claims 1 to 3.

5. Fungal cell of the genus *Trichoderma* comprising a nucleic acid molecule a) or the nucleic acid molecules b) according to any one of claims 1 to 3 or a vector or set of vectors according to claim 4.

6. Fungal cell according to claim 5, **characterized in that** the fungal cell of the genus *Trichoderma* is a *Trichoderma reesei* cell.

7. Fungal cell according to claim 5 or 6, **characterized in that** it comprises at least one nucleic acid molecule whose nucleic acid sequence encodes an N-acetylglucosamine 2-epimerase and an N-acetylneuraminic acid synthase and is operably linked to a constitutive promoter that is active in fungal cells.

8. A method for producing N-acetylneuraminic acid (NeuNAc) comprising cultivating fungal cells of the genus *Trichoderma* according to any one of claims 5 to 7 in the presence of an N-acetyl-D-glucosamine source.

9. Method according to claim 8, **characterized in that** the N-acetyl-D-glucosamine source is chitin.

## Revendications

1. a) Molécule isolée d'acide nucléique comprenant au moins un promoteur actif dans les cellules fongiques du genre Trichoderma, à chacun desquels sont fonctionnellement liées une molécule d'acide nucléique codant pour une N-acétylglucosamine-2-épimérase (EC 5.1.3.8) et une acide N-acétylneuraminique-synthase (EC 2.5.1.56), ou b) ensemble isolé comportant deux molécules d'acides nucléiques, une molécule codant pour la N-acétylglucosamine-2-épimérase (EC 5.1.3.8) et une molécule pour l'acide N-acétylneuraminique-synthase (EC 2.5.1.56), comprenant chacune au moins un promoteur actif dans les cellules fongiques du genre Trichoderma, lié fonctionnellement à la séquence codant pour la N-acétylglucosamine-2-épimérase ou l'acide N-acétylneuraminique-synthase ;
l'au moins un promoteur selon a) ou b), actif dans les cellules fongiques, étant un promoteur constitutif.

2. Molécule d'acide nucléique ou ensemble selon la revendication 1, **caractérisée en ce que** les cellules fongiques du genre Trichoderma sont des cellules de Trichoderma reesei.

3. Molécule d'acide nucléique ou ensemble selon la revendication 1 ou 2, **caractérisée en ce que** le promoteur constitutif est choisi dans le groupe consistant en les promoteurs des gènes de la glycolyse, en particulier pki, gpd ou zwf1, tef1a, act, cox4, neg1 et sar1.

4. Vecteur comprenant une molécule d'acide nucléique a) selon l'une des revendications 1 à 3 ou ensemble de vecteurs comprenant les molécules d'acides nucléiques b) selon l'une des revendications 1 à 3.

5. Cellule fongique du genre Trichoderma, comprenant une molécule d'acide nucléique a) ou les molécules d'acides nucléiques b) selon l'une des revendications 1 à 3 ou un vecteur ou un ensemble de vecteurs selon la revendication 4.

6. Cellule fongique selon la revendication 5, **caractérisée en ce que** la cellule fongique du genre Trichoderma est Trichoderma reesei.

7. Cellule fongique selon la revendication 5 ou 6, **caractérisée en ce qu'**elle comprend au moins une molécule d'acide nucléique dont la séquence d'acide nucléique code pour une N-acétylglucosamine-2-épimérase et une acide N-acétylneuraminique-synthase et est fonctionnellement liée à un promoteur constitutif actif dans les cellules fongiques.

8. Procédé de fabrication d'acide N-acétylneuraminique (NeuNAc), comprenant la culture de cellules fongiques du genre Trichoderma selon l'une des revendications 5 à 7 en présence d'une source de N-acétyl-D-glucosamine.

9. Procédé selon la revendication 8, **caractérisé en ce que** la source de N-acétyl-D-glucosamine est la chitine.
